# EUROPEAN PATENT APPLICATION

(11) **EP 1 627 629 A1**
(43) Date of publication of application: **22.02.2006**
(21) Application number: 03815732.7
(22) Date of filing: 05.02.2003
(51) Int. Cl.: A61K 9/00, A61K 9/48, A61K 9/02

(54) **SKIN LOTION**

(71) Applicant: Miyoshi Kasei, Inc., Saitama-shi Saitama 336-0975 (JP); ASAHI GLASS COMPANY LTD., Tokyo 100-8405 (JP); Dohkai Chemical Industry Co., Ltd., Kitakyushu-shi, Fukuoka (JP)
(72) Inventor: HORINO, Masaakira, Sagamihara-shi, Kanagawa 229-0011 (JP); MATSUBARA, Toshiya, Ichihara-shi, Chiba 290-0058 (JP); TANAKA, Masaharu, Ichihara-shi, Chiba 290-0058 (JP); MIYOSHI, Taizo, Saitama-shi, Saitama 338-0001 (JP); INOUE, Masaki c/o DOHKAI CHEMICAL INDUSTRY CO.,, Kitakyushu-shi, Fukuoka 808-0027 (JP)
(74) Representative: Quantin, Bruno Marie Henri
(86) International application number: PCT/JP2003/001150
(87) International publication number: WO 2004/069210

(57) **Abstract**

There is provided a cosmetic, particularly a skin cosmetic, excellent in imparting transparency (transparent feeling) to the skin, conditioning the skin texture and making wrinkles and hair follicles imperceptible when applied. The cosmetic comprises a powder of porous spherical silica having an average particle size (based on volume) from 3.0 to 20 µm, a maximum particle size of 50 µm or smaller, and a pore volume from 1.5 to 3.0 cm³/g, wherein the powder is **characterized in that** the minimum value of dlog (storage elasticity) /dlog (shear stress) is -10 or more, when 40 g of squalane is added to 15 cm³ of apparent volume of said powder of porous spherical silica, the resulting paste is charged in thickness of 2 mm between parallel plates of 2.5 cm diameter, one of the plates is subjected to an angular vibration to another in a frequency of 2 Hz, dynamic viscoelasticity is measured by increasing an average shear stress between the plates from 10 Pa to 10 kPa and correlation between log (shear stress) versus log (storage elasticity) is measured.

## Description

### TECHNICAL FIELD

The present invention relates to a cosmetic which comprises a powder of porous spherical silica having a disintegrating property and preferably to a cosmetic suitable for the skin. Particularly, it relates to a skin cosmetic which imparts transparency (transparent feeling) to the skin, conditions the skin texture and makes wrinkles and hair follicles imperceptible when applied.

### BACKGROUND ART

With regard to a powder (an additive) comprised in cosmetics, silica powder is one of the most commonly used substance(s) and various silica powders having different characteristics such as shape, pore size and the like are comprised in cosmetics depending upon the purpose of use.

For example, powders of irregular shaped silica, spherical silica, etc. are typical known examples, however all of them have the following problems.

When a skin cosmetic comprising the powder of silica, whose shape is irregular or not spherical, is applied to the skin, creaky feeling is noted, and spots are easy to be resulted so that uniform and beautiful cosmetic finish is not achieved. As a result, functions (effects) of imparting a transparent feeling to the skin, conditioning the skin texture and making wrinkles and hair follicles imperceptible are not achieved. When powder of spherical silica having no disintegrating property is comprised in skin cosmetic, although such cosmetic is excellent in smoothness on the skin, there are disadvantages that, due to its spherical shape, the cosmetic is apt to come off from the skin with a lapse of time and also to be accumulated on skin grooves, not spreading uniformly on the skin, whereby wrinkles are emphasized and makeup is easy to come off. Accordingly, it is not possible to achieve the function (effect) of imparting transparency to the skin, conditioning the skin texture and making wrinkles and hair follicles imperceptible with the above mentioned powder.

On the other hand, with regard to powder of porous spherical silica having disintegrating property, there has been no report for use as a cosmetic thereof and for effect thereof.

Therefore, there is much to be desired for excellent cosmetic powder without above-describe problems.

### DISCLOSURE OF THE INVENTION

### 1. Problems of the Invention

Surface of human skin has color and its structure is not a simple convex shape. Its structure and property is specific having many grooves with an average depth of about 10 µm, and the rough texture as such disturbs a beautiful makeup effect. Especially by aging, grooves on the skin surface become deeper, and this makes a shade in light and distinguishes wrinkles and hair follicles. Accordingly, there has been a demand for development of cosmetic powder by which surface of human skin as such is able to be improved.

Under such circumstances, the problem to be solved by the present invention is to provide a cosmetic or, preferably, a skin cosmetic comprising powder which imparts transparency, conditions skin texture and makes wrinkles and hair follicles imperceptible.

### 2. Means for Solving the Problems

The present inventors have conducted intensive investigations for solving the above problems and found that, when a powder of porous and spherical silica having disintegrating property (disintegrable porous spherical silica powder) is comprised in a cosmetic as a powder for cosmetics, preferably, a cosmetic for skin, it has functions of imparting transparency to the skin, conditioning the skin texture and making wrinkles and hair follicles imperceptible.

It has been also found that an aimed disintegrable porous spherical silica powder can be obtained by selecting a powder, which shows the minimum value of dlog (storage elasticity) /dlog (shear stress) is -10 or more, from porous spherical silica powders having an average particle size (based on volume) from 3.0 to 20 µm, a maximum particle size of 50 µm or less, and a pore volume from 1.5 to 3.0 cm³/g, according to the following process. 40 g of squalane is added to 15 cm³ of apparent volume of the powders of porous spherical silica. The resulting paste is charged in thickness of 2 mm between parallel plates of 2. 5 cm diameter, and then one of the plates is subjected to an angular vibration to another in a frequency of 2 Hz. Dynamic viscoelasticity is measured by increasing an average shear stress between the plates from 10 Pa to 10 kPa, and correlation between log (shear stress) versus log (storage elasticity) is measured.

Thus, the present invention relates to a cosmetic, preferably a skin cosmetic, which comprises a powder of porous spherical silica having an average particle size (based on volume) from 3.0 to 20 µm, a maximum particle size of 50 µm or smaller, and a pore volume from 1.5 to 3.0 cm³/g, wherein the powder is characterized in that, the minimum value of dlog (storage elasticity)/dlog (shear stress) is -10 or more when 40 g of squalane is added to 15 cm³ of apparent volume of the powder of porous spherical silica, the resulting paste is charged in thickness of 2 mm between parallel plates of 2.5 cm diameter, one of the plates is subjected to an angular vibration to another in a frequency of 2 Hz, dynamic viscoelasticity is measured by increasing an average shear stress between the plates from 10 Pa to 10 kPa and correlation between log (shear stress) versus log (storage elasticity) is measured.

It is preferred in the present invention that the cosmetic contains the above porous spherical silica powder in amounts of about 1 to 80% by mass.

In the present invention, when the cosmetic containing the disintegrable porous spherical silica powder is applied to the face or the skin, the powder is gradually disintegrated from the particle surface by abrasion on the surface of the face and makes the skin surface in a uniform height and, since refractive index is 1.45 which is lower than the skin, there is achieved an effect that roughness of wrinkles, hair follicles and skin texture is made imperceptible. In addition, by incorporating ground nutrition protector, sebum and moisture into the particles to make the cosmetic transparent, to the cosmetic can be used as a cosmetic having better transparency (transparent feeling) and presenting bare skin feeling.

### MODE FOR CARRYING OUT THE INVENTION

Mode for carrying out the present invention will be illustrated as hereunder.

With regard to production of the disintegrable porous spherical silica powder used in the present invention, the powder can, for example, be produced by the following process.

Firstly, an aqueous solution of alkali silicate is emulsified in a non-polar organic halide solvent containing a surfactant and then a gelling agent is added thereto so that the spherical silica is gelled to prepare spherical silica gel.

Although there is no particular limitation for the alkali silicate, advantageously used ones are sodium silicate, potassium silicate, etc. With regard to the concentration of silica in the aqueous solution of alkali silicate, about 5 to 25% by mass is preferred. When the concentration is less than 5% by mass, productivity is low and that is not preferred in view of economy. When the concentration is more than 25% by mass, rigid gel is produced and aimed pore volume is hardy achieved whereby that is not preferred. More preferably, it is about 5 to 15% by mass. Further, when a water-soluble inorganic salt such as sodium chloride is previously added to an aqueous solution of alkali silicate and then gelling is conducted, more rough gel is produced and that is very convenient in view of achievement of aimed pore volume. With regard to the non-polar organic halide solvent, preferably used ones are 2,2-dichloro-1,1,1-trifluoroethane (HCFC-123), 1,1-dichloro-1-fluoroethane (HCFC-141b), 1,2,2,3,3-pentafluoro-1,1-dichloropropane (R-225cb), methylene chloride, etc. and it is particularly preferred to use 2,2-dichloro-1,1,1-trifluoroethane (HCFC-123) or 1,1-dichloro-1-fluoroethane (HCFC-141b).

With regard to the surfactant, preferably used ones are polyethylene glycol fatty acid ester, polyoxyethylene alkyl phenyl ether, polyoxyethylene alkyl ether, sorbitan fatty acid ester, polyoxyethylene sorbitan fatty acid ester, etc.

With regard to the gelling agent, it is preferred to use an acid. With regard to the acid, inorganic acid is preferred and, although carbon dioxide gas, boric acid, etc. are used, carbon dioxide gas is used particularly preferably.

The above silica gel spheres are obtained as an aqueous slurry containing silica gel spheres and the aqueous slurry is separated from a non-polar organic halide solvent utilizing the difference in density and then aged for a predetermined period under the condition of predetermined pH and temperature.

A preferred aging condition for the production of disintegrable (shear brittle) porous spherical silica powder in the invention is that pH is about 0.5 to 2.5 and temperature is about 60 to 90°C. After aging, the slurry is separated into solid and liquid and the cake is washed with water and dried to obtain powder of porous spherical silica.

Although there is no particular limitation for the above solid-liquid separation method and drying method, centrifugal separator, compressed filter, vacuum filter, etc. are preferably used for a solid-liquid separation method while, for a drying method, air flow drier, rotary drier, aeration band drier, etc. are preferably used.

With regard to the aimed powder of porous spherical silica (particles) in the present invention, it is necessary to firstly adjust to such an extent that average particle size on the basis of volume is about 3.0 to 20 µm, maximum particle size is 50 µm or smaller and pore volume is about 1.5 to 3.0 cm³/g.

As mentioned above, with regard to the above average particle size of the aimed porous spherical silica (particle) powder, it is adjusted to be about 3.0 to 20 µm. When the average particle size is less than 3.0 µm, surface of the particles is hardly disintegrated and disintegrating ability is not sufficient whereby that is not preferred. On the other hand, when an average particle size is more than 20 µm, particle surface is apt to be crumbled and is disintegrated during a mixing step for compounding of cosmetic and during a molding step and a sufficient usability is not achieved whereby that is not preferred. Preferred range of the average particle size is about 4.0 to 15 µm. Here, the average particle size is able to be measured by a laser scattering method.

In addition, as mentioned above, maximum particle size of the above-mentioned aimed porous spherical silica (particle) powder is to be made 50 µm or smaller. When the maximum particle size is more than 50 µm, uncomfortable feeling and rough feeling are strong when applied to the skin and that is not preferred. In view of achieving a good feeling in use without uncomfortable feeling and rough feeling, it is preferred that the maximum particle size is within a range of about 30 to 45 µm.

With regard to the pore volume of the above aimed porous silica (particle) powder, it is adjusted to about 1.5 to 3.0 cm³/g as mentioned above. When the pore volume is less than 1. 5 cm³/g, particles are not disintegrated and a disintegrating property is not sufficient whereby that is not preferred. When the pore volume is more than 3.0 cm³/g, absorbing ability for sebum and moisture is excessive and dry feeling of the skin becomes too strong and, therefore, the feeling in use becomes significantly bad whereby that is not preferred. More preferred range for the pore volume is about 1.7 to 2.5 cm³/g. Incidentally, pore volume is able to be measured by a nitrogen absorption/desorption method.

With regard to the aimed porous spherical silica (particle) powder in the present invention, when a paste prepared by addition of squalane to the porous spherical silica powder is subjected to a measurement for dynamic viscoelasticity and the correlation between log (shear stress) versus log (storage elasticity) is determined, the minimum value of negative gradient (dlog (storage elasticity)/dlog (shear stress)) when the storage elasticity decreases is necessary to be -10 or more or, preferably, -8.0 or more.

Dynamic viscoelasticity can be measured with the following preferable method.

Firstly, 40 g of squalane is added to 15 cm³ (in terms of apparent volume) of the porous spherical silica powder and well mixed to prepare a paste. The paste is charged between two parallel disks (diameter: 2.5 cm) of a measuring device for viscoelasticity of a parallel-plate-type so as to make the distance between the plates 2 mm. One of the above plates is subjected an angular vibration in a frequency of 2 Hz to another plate to apply a periodical shear stress to the paste whereupon its viscoelasticity is dynamically evaluated. The storage elasticity is measured by a stepwise increase of an average shear stress between the plates from 10 Pa to 10 kPa. Since the shear stress changes in a form of sine curve, the maximum value in the periodical change is adopted as the value of the shear stress. The term "Average" of the average shear stress means an average of the shear stress in the plane of the plate. Hereinafter in the present specification, such a measuring method may be just referred to as "a measuring method for dynamic viscoelasticity".

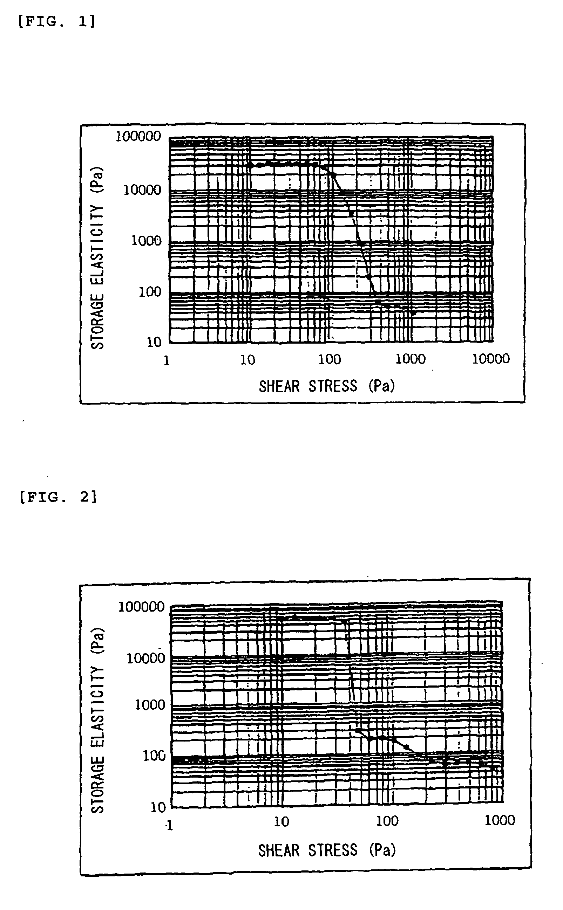

Figs. 1 to 3 are examples showing the correlation between log (shear stress) versus log (storage elasticity). In the above-mentioned paste, there is an area where the storage elasticity greatly decreases when the shear stress becomes large. In the aimed porous spherical silica powder in the present invention, the minimum value for dlog (storage elasticity) /dlog (shear stress) is -10 or more in the changing area as such.

With respect to a cosmetic such as milky lotion, body cream, scrubbing agent and cleansing cream, analysis of behavior of dynamic viscoelasticity has been conducted for a purpose of a quantitative grasp for usability when such a cosmetic is applied to the skin. To be more specific, when the test sample is loaded together with vibration, and frequency of the vibration is increased, components such as compounded particles begin to move as the shear stress increases and, at the same time, storage elasticity decreases accordingly. When the decrease in the storage elasticity is slow or, in other words, when the minimum value of the gradient value (dlog (storage elasticity)/dlog (shear stress)) as the level of storage elasticity lowers is large, there is a tendency that resistance upon application is little and smooth whereupon an applying feeling with a good adhesion is achieved. In the disintegrable spherical porous silica powder used in the present invention, the minimum value of the gradient value (dlog (storage elasticity)/dlog (shear stress)) is large when the level of storage elasticity lowers and, although the powder is not broken by a mechanical shock in the steps for the manufacture of cosmetic, the powder has a property of being easily disintegrated by shear stress (pressure), abrasion, etc. by finger, puff or the like, and, as the shear stress increases, gradual disintegration starts from the particle surface and expands whereby it is likely that such a result is achieved.

From the above, it may be concluded with regard to the disintegrable porous spherical silica powder used in the cosmetic of the present invention that, although resistance is little and movement is smooth in the applying stage (initial stage) of the cosmetic, particle surface is gradually disintegrated as the application is expanded whereby it is fixed to the skin being closely adhered thereto and, as a result, functions of imparting transparency to the skin, conditioning the skin texture and making wrinkles and hair follicles imperceptible are able to be achieved.

In the present invention, there is no particular limitation for product form and shape of the cosmetic comprising the above disintegrable porous spherical silica powder, and the examples thereof are powdery, pressed, liquid and stick-shaped products, and examples of liquid type ones are cosmetic of an emulsified type and an oily type. Specific examples are cosmetics such as face powder, foundation, pressed powder, eye shadow, lip color, lip glow, eye liner, mascara, eye blow, foundation cream and powder-containing lotion.

The amount (ratio) of the comprised disintegrable porous spherical silica powder in the cosmetic according to the present invention may be selected depending upon dosage form, purpose of use, etc. of the cosmetic and there is no particular limitation therefor. Preferably, about 1 to 80% by mass is selected, more preferably, about 2 to 70% by mass is selected and, still more preferably, about 5 to 65% by mass is selected. When the comprised amount is less than 1% by mass, it is hard to achieve the function of imparting transparency to the skin, conditioning the skin texture and making wrinkles and pores imperceptible and, therefore, that is not preferred. When the comprised amount is more than 80% by mass, the feeling becomes powdery and rustling feeling becomes strong causing changes in the usability of the cosmetic and, therefore, that is not preferred.

In addition to the above-mentioned disintegrable porous spherical silica powder which is an essential ingredient, the cosmetic of the present invention may be further comprised or mixed, depending upon product type, purpose of use, etc., with fat/oil (oily agent) or wax such as paraffin, ceresin, liquid paraffin, castor oil, Japan wax, lanoline, beeswax, carnauba wax, candelila wax, plant oil, plant oil ester, fatty acid, higher alcohol and squalane; surfactants such as anionic surfactant (e.g., sodium alkyl sulfate), cationic surfactant (e.g., alkyldimethylammonium betaine), amphoteric surfactant (e.g., alkyltrimethylammonium chloride) and nonionic surfactant (e.g., polyoxyethylene alkyl ether); moisturizer such as polyhydric alcohol and propylene glycol; resin; dispersing agent; dye; perfume; antiseptic agent; pharmaceutical component; coloring pigment; inorganic powder; organic powder; solvent; and various kinds of other additives which have been commonly used as materials for cosmetics.

It is also possible that the above components such as additive and oily agent, which are able to be comprised in the cosmetic, are previously contained in small pores of the above-prepared disintegrable porous spherical silica powder and that the resulting powder is comprised in the cosmetic.

In the present invention, it is possible that particle surface of the above disintegrable porous spherical silica powder (particle) is treated and comprised in the cosmetic.

For example, the surface is coated with silicone oil, silane coupling agent, fluorine-type agent for making hydrophobic/lipophobic, titanate coupling agent, alcohol, surfactant and other surface treating agent or surface improving agent to give a surface-treated powder, etc. and the resulting powder is able to be comprised in a cosmetic or, preferably, with a skin cosmetic. Such a powder treated with a surface treating agent or a surface improving agent, whereby the surface is made hydrophobic, exhibits an excellent sustained cosmetic effect when comprised in a cosmetic.

Skin cosmetic containing disintegrable porous spherical silica powder which is subjected to a surface treatment with a hydrophobizing agent such as methyl hydrogen polysiloxane and dimethylsilicone having a reactive group in a molecule or a hydrophilizng/hydrophobizing agent of a fluorine type such as perfluoroalkyl silane is non-sticky, imparts smooth touch and has an excellent spread on the skin (such as face) at the stage of being applied (at the initial stage of application of the cosmetic). In addition, the powder is gradually disintegrated from the particle surface by abrasion, etc. on the skin surface whereby a skin cosmetic having non-dry (moist), mild and good fitting feel (closely contacting feeling) is able to be available. Further, in the case of a skin cosmetic containing the disintegrable porous spherical silica powder subjected to the surface treatment, although specific surface area of the disintegrable porous spherical silica powder is as large as 600 to 800 m²/g, it gradually absorbs excessive sebum and moisture and therefore, there is no dry feeling or uncomfortable feeling on the skin and a sustained cosmetic effect is good.

### BRIEF DESCRIPTION OF THE DRAWINGS

Fig. 1 is a graph which shows the correlation of log (shear stress) versus log(storage elasticity) measured for the disintegrable porous spherical silica (particle) powder prepared in Manufacturing Example 1 concerning the present invention.
Fig. 2 is a graph which shows the correlation of log (shear stress) versus log (storage elasticity) measured for the porous spherical silica (particle) powder prepared in Manufacturing Example 2 for comparison.
Fig. 3 is a graph which shows the correlation of log (shear stress) versus log (storage elasticity) measured for the porous spherical silica (particle) powder prepared in Manufacturing Example 3 for comparison.

### PREFERRED MODE FOR CARRYING OUT THE INVENTION

The present invention will now be illustrated in detail by way of the following Manufacturing Examples, Examples, Comparative Examples and Sensory Evaluation Examples. They are to make the technical significance of the present invention clearer and the scope of the present invention shall not be interpreted in a limited manner by those.

### (Manufacturing Example 1)

Sodium silicate No. 3 (Sodium Silicate of JIS K 1408-66 No. 3) (50 g) containing 29.0% by mass of SiO₂ and 4.0 g of sodium chloride were dissolved in 91.0 g of deionized water to prepare an aqueous solution of sodium silicate containing 2.76% by mass of sodium chloride where SiO₂ concentration was 10.0% by mass. After that, the above-prepared aqueous solution of sodium silicate was added to 240 cm³ of HCFC-123, in which 0.84 g of sorbitan monooleate was dissolved (surfactant concentration: 3,500 ppm) with stirring at 5,000 rpm using a stirrer (Auto Homo-Mixer manufactured by Tokushu Kika Kogyo) and the mixture was stirred for 5 minutes. Then carbon dioxide gas was blown thereinto at a flow rate of 200 cm³/minute for 15 minutes under the temperature condition of 10°C to conduct gelling. The resulting gel was separated from HCFC-123, adjusted to pH 2.0 by addition of sulfuric acid of 20.0% by mass concentration, aged at 80°C for 1 hour and further subjected to a solid-liquid separation using a vacuum filter and the cake was washed with 4,000 cm³ of water and dried at 300°C using an air-flow dryer to give powder of disintegrable porous spherical silica (particles).

When an average particle size of the resulting silica powder was measured by a Coulter counter (manufactured by Nikkaki), it was 4.1 µm. When particles of the powder were observed under a scanning electron microscope, the maximum particle size was about 40 µm. Pore volume measured by Omnisorp (manufactured by Omicron) was 2.0 cm³/g.

To 15 cm³ of the powder was added 40 g of squalane to prepare a slightly hard paste and its dynamic viscoelasticity was measured under the condition of 2 Hz at room temperature using a universal rheometer of a DAR type (manufactured by Rheologica). Fig. 1 shows a correlation of dlog (storage elasticity)/dlog (shear stress) by a graph. As a result, storage elasticity decreased from 27916 to 61 Pa within stress values of 79.4 to 375.3 Pa and the minimum value of dlog (storage elasticity)/dlog (shear stress) was about -3.9.

### (Manufacturing Example 2)

Sodium silicate No. 3 (50 g) containing 24.0% by mass of SiO₂ was dissolved in 10.0 g of deionized water to prepare an aqueous solution where SiO₂ concentration was 20.0% by mass. After that, the above-prepared aqueous solution of sodium silicate was added to 240 cm³ of HCFC-123, in which 0.84 g of sorbitan monooleate was dissolved (surfactant concentration: 3,500 ppm) with stirring at 5,000 rpm using a stirrer (Auto Homo-Mixer manufactured by Tokushu Kika Kogyo) and the mixture was stirred for 5 minutes. Then carbon dioxide gas was blown thereinto at a flow rate of 200 cm³/minute for 15 minutes under the temperature condition of 10°C to conduct gelling. The resulting gel was separated from HCFC-123, adjusted to pH 2.0 by addition of sulfuric acid of 20.0% by mass concentration, aged at 80°C for 1 hour and further subjected to a solid-liquid separation using a vacuum filter and the cake was washed with 1, 500 cm³ of water and dried at 300°C using an air-flow dryer to give powder of porous spherical silica (particles).

When an average particle size of the resulting silica powder was measured by a Coulter counter (manufactured by Nikkaki), it was 4.9 µm. When particles of the powder were observed under a scanning electron microscope, the maximum particle size was about 40 µm. Pore volume measured by Omnisorp (manufactured by Omicron) was 0.9 cm³/g.

To 15 cm³ of the powder was added 40 g of squalane to prepare a slightly hard paste and its dynamic viscoelasticity was measured under the condition of 2 Hz at room temperature using a universal rheometer of a DAR type (manufactured by Rheologica). Fig. 2 shows a correlation of dlog (storage elasticity)/dlog (shear stress) by a graph. As a result, storage elasticity decreased from 44773 to 268 Pa within stress values of 36.5 to 47.3 Pa and the minimum value of dlog (storage elasticity)/dlog (shear stress) was about -19.7.

### (Manufacturing Example 3)

Sodium silicate No. 3 (50.0 g) containing 29.0% by mass of SiO₂ and 4.0 g of sodium chloride were dissolved in 91.0 g of deionized water to prepare an aqueous solution of sodium silicate containing 2.76% by mass of sodium chloride where SiO₂ concentration was 10.0% by mass. After that, the above-prepared aqueous solution of sodium silicate was added to 240 cm³ of HCFC-123, in which 0.84 g of sorbitan monooleate was dissolved (surfactant concentration: 3,500 ppm) with stirring at 8,000 rpm using a stirrer (Auto Homo-Mixer manufactured by Tokushu Kika Kogyo) and the mixture was stirred for 5 minutes. Then carbon dioxide gas was blown thereinto at a flow rate of 200 cm³/minute for 15 minutes under the temperature condition of 10°C to conduct gelling. The resulting gel was separated from HCFC-123, adjusted to pH 2.0 by addition of sulfuric acid of 20.0% by mass concentration, aged at 80°C for 1 hour and further subjected to a solid-liquid separation using a vacuum filter and the cake was washed with 4, 000 cm³ of water and dried at 300°C using an air-flow dryer to give powder of porous spherical silica (particles).

When an average particle size of the resulting silica powder was measured by a Coulter counter (manufactured by Nikkaki), it was 2.5 µm. When particles of the powder were observed under a scanning electron microscope, the maximum particle size was about 30 µm. Pore volume measured by Omnisorp (manufactured by Omicron) was 2.1 cm³/g.

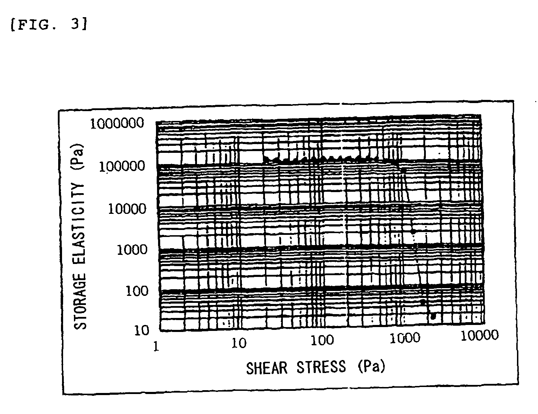

To 15 cm³ of the powder was added 40 g of squalane to prepare a slightly hard paste and its dynamic viscoelasticity was measured under the condition of 2 Hz at room temperature using a universal rheometer of a DAR type (manufactured by Rheologica). Fig. 3 shows a correlation of dlog (storage elasticity)/dlog (shear stress) by a graph. As a result, storage elasticity decreased from 55673 to 36 Pa within stress values of 1057 to 1775 Pa and the minimum value of dlog (storage elasticity)/dlog (shear stress) was about -14.2.

### (Manufacturing Example 4)

Sodium silicate No. 3 (50.0 g) containing 24.0% by mass of SiO₂ was dissolved in 16.7 g of deionized water to prepare an aqueous solution where SiO₂ concentration was 18.0% by mass. After that, the above-prepared aqueous solution of sodium silicate was added to 240 cm³ of HCFC-123, in which 0.84 g of sorbitan monooleate was dissolved (surfactant concentration: 3,500 ppm) with stirring at 1,600 rpm using a stirrer (Auto Homo-Mixer manufactured by Tokushu Kika Kogyo) and the mixture was stirred for 5 minutes. Then carbon dioxide gas was blown thereinto at a flow rate of 200 cm³/minute for 15 minutes under the temperature condition of 10°C to conduct gelling. The resulting gel was separated from HCFC-123, adjusted to pH 2.0 by addition of sulfuric acid of 20.0% by mass concentration, aged at 80°C for 1 hour and further subjected to a solid-liquid separation using a vacuum filter and the cake was washed with 1, 500 cm³ of water and dried at 300°C using an air-flow dryer to give powder of porous spherical silica (particles).

When an average particle size of the resulting silica powder was measured by a Coulter counter (manufactured by Nikkaki), it was 25.0 µm. When particles of the powder were observed under a scanning electron microscope, the maximum particle size was about 75 µm. Pore volume measured by Omnisorp (manufactured by Omicron) was 1.2 cm³/g.

To 15 cm³ of the powder was added 40 g of squalane to prepare a slightly hard paste and its dynamic viscoelasticity was measured under the condition of 2 Hz at room temperature using a universal rheometer of a DAR type (manufactured by Rheologica). Storage elasticity decreased from 23850 to 21 Pa within stress values of 28.1 to 61.3 Pa and the minimum value of dlog (storage elasticity)/dlog (shear stress) was about -9.0.

### (Manufacturing Example 5)

Sodium silicate No. 3 (50.0 g) containing 29.0% by mass of SiO₂ and 3.5 g of sodium chloride were dissolved in 84.6 g of deionized water to prepare an aqueous solution of sodium silicate containing 2.53% by mass of sodium chloride where SiO₂ concentration was 10.5% by mass. After that, the above-prepared aqueous solution of sodium silicate was added to 240 cm³ of HCFC-123, in which 0.84 g of sorbitan monooleate was dissolved (surfactant concentration: 3,500 ppm) with stirring at 2,000 rpm using a stirrer (Auto Homo-Mixer manufactured by Tokushu Kika Kogyo) and the mixture was stirred for 5 minutes. Then carbon dioxide gas was blown thereinto at a flow rate of 200 cm³/minute for 15 minutes under the temperature condition of 10°C to conduct gelling. The resulting gel was separated from HCFC-123, adjusted to pH 2.0 by addition of sulfuric acid of 20.0% by mass concentration, aged at 80°C for 1 hour and further subjected to a solid-liquid separation using a vacuum filter and the cake was washed with 4,000 cm³ of water and dried at 300°C using an air-flow dryer to give powder of disintegrable porous spherical silica (particles).

When an average particle size of the resulting silica powder was measured by a Coulter counter (manufactured by Nikkaki), it was 18.0 µm. When particles of the powder were observed under a scanning electron microscope, the maximum particle size was about 48 µm. Pore volume measured by Omnisorp (manufactured by Omicron) was 1.6 cm³/g.

To 15 cm³ of the powder was added 40 g of squalane to prepare a slightly hard paste and its dynamic viscoelasticity was measured under the condition of 2 Hz at room temperature using a universal rheometer of a DAR type (manufactured by Rheologica). Storage elasticity decreased from 32450 to 95 Pa within stress values of 36.5 to 79.4 Pa and the minimum value of dlog (storage elasticity)/dlog (shear stress) was about -7.5.

### (Manufacturing Example 6)

Sodium silicate No. 3 (45.0 g) containing 29.0% by mass of SiO₂ and 5.0 g of sodium chloride were dissolved in 95.0 g of deionized water to prepare an aqueous solution of sodium silicate containing 3.45% by mass of sodium chloride where SiO₂ concentration was 9.0% by mass. After that, the above-prepared aqueous solution of sodium silicate was added to 240 cm³ of HCFC-123, in which 0.84 g of sorbitan monooleate was dissolved (surfactant concentration: 3,500 ppm) with stirring at 7,000 rpm using a stirrer (Auto Homo-Mixer manufactured by Tokushu Kika Kogyo) and the mixture was stirred for 5 minutes. Then carbon dioxide gas was blown thereinto at a flow rate of 200 cm³/minute for 15 minutes under the temperature condition of 10°C to conduct gelling. The resulting gel was separated from HCFC-123, adjusted to pH 2.0 by addition of sulfuric acid of 20.0% by mass concentration, aged at 80°C for 1 hour and further subjected to a solid-liquid separation using a vacuum filter and the cake was washed with 4,000 cm³ of water and dried at 300°C using an air-flow dryer to give powder of porous spherical silica (particles).

When an average particle size of the resulting silica powder was measured by a Coulter counter (manufactured by Nikkaki), it was 3.2 µm. When particles of the powder were observed under a scanning electron microscope, the maximum particle size was about 33 µm. Pore volume measured by Omnisorp (manufactured by Omicron) was 2.7 cm³/g.

To 15 cm³ of the powder was added 40 g of squalane to prepare a slightly hard paste and its dynamic viscoelasticity was measured under the condition of 2 Hz at room temperature using a universal rheometer of a DAR type (manufactured by Rheologica). Storage elasticity decreased from 52352 to 42 Pa within stress values of 486 to 1775 Pa and the minimum value of dlog (storage elasticity)/dlog (shear stress) was about -5.5.

### (Example 1)

Using the disintegrable porous spherical silica powder prepared in Manufacturing Example 1, a powder foundation was manufactured by compounding of predetermined amounts of material components by the following method.

Firstly, the material components (7) to (12) were mixed with stirring by a Henshel mixer and ground using an atomizer.

Then a material component (6) was added to the above-ground material component mixture and mixed with stirring using a Henshel mixer, the material components (1) to (5) which were previously heated, melted and mixed were added thereto and the mixture was mixed with stirring using a Henshel mixer and ground by an atomizer.

The above-ground material mixture was molded by compression using a metal mold to prepare a powder foundation.

Evaluation was conducted for the resulting powder foundation and spread upon application was good, touch was smooth and closely adhesion property was excellent. Effect of conditioning the skin texture, transparency and effect for making pores imperceptible were excellent as well and cosmetic membrane upon finish was uniform and natural (refer to Table 1 which will be mentioned later).

| Components | Composition (mass %) |
|---|---|
| (1) Squalane | 8.6 |
| (2) Silicone oil (100 cs) | 12.0 |
| (3) Vaseline | 0.67 |
| (4) Glycerol | 0.67 |
| (5) Perfume | 0.06 |
| (6) Silica powder manufactured in Manufacturing Example 1 | 33.0 |
| (7) Silicon-treated talc | 15.0 |
| (8) Silicon-treated titanium oxide | 10.0 |
| (9) Silicon-treated yellow iron oxide | 1.0 |
| (10) Silicon-treated red iron oxide | 0.8 |
| (11) Silicon-treated black iron oxide | 0.06 |
| (12) Silicon-treated sericite | 18.14 |

### (Comparative Example 1)

Powder foundation was manufactured using the same material components and compounding (manufacturing) condition as those in Example 1 except that the porous spherical silica powder prepared in Manufacturing Example 2 was used (as silica powder) instead of silica powder prepared in Manufacturing Example 1.

The result of evaluation for the resulting powder foundation was that, although spread upon application was good and the touch was smooth, the porous spherical silica powder prepared in Manufacturing Example 2 has no disintegrating property and, therefore, a sufficient closely adhesive feel is not achieved and, with regard to effect for conditioning the skin texture, transparent feeling and effect for making pores imperceptible, they are inferior to those of the powder foundation prepared in Example 1 (refer to Table 1 which will be mentioned later).

### (Comparative Example 2)

Powder foundation was manufactured using the same material components and compounding (manufacturing) condition as those in Example 1 except that the porous spherical silica powder prepared in Manufacturing Example 3 was used (as silica powder) instead of silica powder prepared in Manufacturing Example 1.

The result of evaluation for the resulting powder foundation was that, although spread upon application was good and the touch was smooth, the porous spherical silica powder prepared in Manufacturing Example 3 has no disintegrating property and, therefore, a sufficient closely adhesive feel is not achieved and, with regard to effect for conditioning the skin texture, transparent feeling and effect for making pores imperceptible, they are inferior to those of the powder foundation prepared in Example 1 (refer to Table 1 which will be mentioned later).

### (Comparative Example 3)

Powder foundation was manufactured using the same material components and compounding (manufacturing) condition as those in Example 1 except that the porous spherical silica powder prepared in Manufacturing Example 4 was used (as silica powder) instead of silica powder prepared in Manufacturing Example 1.

The result of evaluation for the resulting powder foundation was that, since particle size of the porous spherical silica powder prepared in Manufacturing Example 4 was too big, spread upon application was poor giving some unconformable feeling, smooth touch and closely adhering property were not fully achieved and usability was inferior. In addition, due to the same reason, effect for conditioning the skin texture, transparent feeling and effect for making pores imperceptible were inferior to the powder foundation prepared in Example 1 (refer to Table 1 which will be mentioned later).

### (Sensory Evaluation Example 1)

In order to evaluate the powder foundation prepared in each of the above Example 1 and Comparative Examples 1, 2 and 3, an sensory evaluation was conducted by 20 female panelists for spread (extension), effect for conditioning the skin texture, transparency and effect for making hair follicles imperceptible. The result is shown in Table 1. The evaluation was conducted by a five-point method and the average mark was adopted.
- 5: very good
- 4: somewhat good
- 3: normal
- 2: somewhat inferior
- 1: much inferior

**[Table 1] Sensory Evaluation for Powder Foundations**

| Samples | Spread | Skin Texture ^{*1} | Transparency | Hair follicles ^{*2} |
|---|---|---|---|---|
| Example 1 | 4.20 | 3.90 | 4.20 | 3.75 |
| Comp Ex 1 | 4.55 | 2.35 | 1.95 | 2.15 |
| Comp Ex 2 | 3.25 | 2.45 | 2.00 | 1.75 |
| Comp Ex 3 | 2.30 | 1.20 | 2.00 | 1.85 |

| | | | | |
|---|---|---|---|---|
| *1: Effect for conditioning the skin texture | | | | |
| *2: Effect for making hair follicles imperceptible | | | | |

From the above result, it is noted that, with regard to powder foundation, product of the present invention is better than the conventional products.

### (Example 2)

A mixture of powder components (6) to (12) prepared in Example 1 was used and an oily foundation was manufactured by compounding the material components in predetermined amounts according to the following method.

The material components (1) to (4) were heated to melt, the material component (5) was added to the resulting material component mixture followed by well stirring and mixing using a Henshel mixer and subjecting to a vacuum defoaming by keeping at high temperature and the resulting mixture was charged in a metal plate and solidified by cooling to prepare an oily foundation.

Result of evaluation for the resulting oily foundation is that spread upon application was good imparting a smooth feeling. In addition, the product showed no oiliness but was simple, had a closely adhering property, conditioned the skin texture, gave transparency and made pores imperceptible whereby the cosmetic effect was excellent (refer to Table 2 which will be mentioned later).

| Components | Composition (mass %) |
|---|---|
| (1) Silicone oil (20 cs) | 22.0 |
| (2) Liquid paraffin | 40.0 |
| (3) Vaseline | 2.7 |
| (4) Dextrin palmitate | 5.3 |
| (5) Mixture of powdery components (6) to (12) prepared in Example 1 | 30.0 |

### (Comparative Example 4)

Oily foundation was manufactured using the same material components and compounding (manufacturing) condition as those in Example 2 except that the porous spherical silica powder prepared in Manufacturing Example 2 was used (as silica powder) instead of silica powder prepared in Manufacturing Example 1.

The result of evaluation for the resulting oily foundation was that, although spread upon application was good and the touch was smooth, the porous spherical silica powder used for the manufacture of powdery foundation in Comparative Example 1 (porous spherical silica powder prepared in Manufacturing Example 2) has no disintegrating property and, therefore, a sufficient closely adhesive feel is not achieved and, with regard to effect for conditioning the skin texture, transparent feeling and effect for making pores imperceptible, they are inferior to those of the oily foundation prepared in Example 2 (refer to Table 2 which will be mentioned later).

### (Sensory Evaluation Example 2)

In order to evaluate the oily foundation prepared in each of the above Example 2 and Comparative Example 4, an sensory evaluation was conducted by 20 female panelists for spread, effect for conditioning the skin texture, transparency and effect for making hair follicles imperceptible. The result is shown in Table 2. The evaluation was conducted by a five-point method and the average mark was adopted.
- 5: very good
- 4: somewhat good
- 3: normal
- 2: somewhat inferior
- 1: much inferior

**[Table 2] Organoleptic Evaluation for Oily Foundations**

| Samples | Spread | Skin Texture ^{*1} | Transparency | Hair follicles ^{*2} |
|---|---|---|---|---|
| Example 2 | 3.70 | 4.40 | 4.00 | 3.70 |
| Comp Ex 4 | 3.60 | 2.70 | 2.85 | 1.90 |

| | | | | |
|---|---|---|---|---|
| *1: Effect for conditioning the skin texture | | | | |
| *2: Effect for making hair follicles imperceptible | | | | |

From the above result, it is noted that, with regard to oily foundation, product of the present invention is better than the conventional product.

### (Example 3)

In order to subject the disintegrable porous spherical silica powder prepared in Manufacturing Example 5 to a silicon treatment, the following material components were compounded (mixed) in predetermined amounts, mixed with stirring using a Henshel mixer and heated at 120°C for 7 hours.

| Components | Composition (mass %) |
|---|---|
| (1) Dimethylsilicone oil (200 cs) | 12.0 |
| (2) Methyl hydrogen polysiloxane | 5.0 |
| (3) Silica powder prepared in Manufacturing Example 5 | 83.0 |

Using the silicon-treated disintegrable porous spherical silica powder prepared as above, a powder foundation was manufactured by compounding of predetermined amounts of material components by the following method.

Firstly, the material components (7) to (12) were mixed with stirring by a Henshel mixer and ground using an atomizer.

Then a material component (6) was added to the above-ground material component mixture and mixed with stirring using a Henshel mixer, the material components (1) to (5) which were previously heated, melted and mixed were added thereto and the mixture was mixed with stirring using a Henshel mixer and ground by an atomizer.

The above-ground material mixture was molded by compression using a metal mold to prepare a powder foundation.

Evaluation was conducted for the resulting powder foundation and spread upon application was better, touch was smooth and closely adhesion property was excellent as compared with the product of Example 1. Effect of conditioning the skin texture, transparency and effect for making pores imperceptible were excellent as well and cosmetic membrane upon finish was uniform and natural.

| Components | Composition (mass %) |
|---|---|
| (1) Squalane | 10.6 |
| (2) Silicone oil (100 cs) | 3.0 |
| (3) Vaseline | 0.67 |
| (4) Glycerol | 0.67 |
| (5) Perfume | 0.06 |
| (6) Silicon-treated disintegrable porous spherical silica powder | 33.0 |
| (7) Silicon-treated talc | 12.0 |
| (8) Silicon-treated titanium oxide | 10.0 |
| (9) Silicon-treated yellow iron oxide | 1.0 |
| (10) Silicon-treated red iron oxide | 0.8 |
| (11) Silicon-treated black iron oxide | 0.06 |
| (12) Silicon-treated sericite | 28.14 |

### (Example 4)

Silica powder prepared by a silicon treatment of the disintegrable porous silica powder prepared in Manufacturing Example 5 was used in Example 3 and a liquid foundation was manufactured according to the following method.

| | Components | Composition (mass %) |
|---|---|---|
| (A) | (1) Cyclomethicone | 12.0 |
| | (2) Volatile oil of emulsified type | 2.0 |
| | (3) Silicon-treated silica powder (Example 3) | 4.0 |
| | (4) Silicon-treated titanium oxide | 5.0 |
| | (5) Silicon-treated red iron oxide cloisonne | 0.7 |
| | (6) Silicon-treated yellow iron oxide | 0.2 |
| | (7) Silicon-treated black iron oxide | 0.1 |
| | (8) Silicon-treated talc | 2.0 |
| (B) | (9) Propyl paraben | 0.2 |
| | (10) Polyoxyethylene lauryl ether | 0.5 |
| (C) | (11) Volatile oil of emulsified type | 18.0 |
| | (12) Dimethylsilicone (50 cs) | 3.0 |
| | (13) Tocopherol acetate | 0.1 |
| | (14) Corn oil | 0.05 |
| (D) | (15) Methyl paraben | 0.2 |
| | (16) Propylene glycol | 8.0 |
| (E) | (17) Pure water | 41.45 |
| | (18) Sodium dehydroacetate | 0.3 |
| | (19) Pantothenyl alcohol | 0.2 |
| | (20) Sodium chloride | 2.0 |

With regard to the above components (A), pigment components (4) to (8) were mixed using a Henshel mixer and ground by an atomizer and other components (1) to (3) were added to the ground mixture followed by uniform mixing.

Each of the above components (B), (C), (D) and (E) was heated to dissolve at 60°C, the above uniformly mixed component (A), the above component (B) and the above component (C) were mixed and, similarly, the above component (D) and the above component (E) were mixed.

Aqueous layer component (a mixture of the components (D) and (E)) was added little by little to an oily layer component (a mixture of the components (A) to (C)) which was stirred using a homogenizer to emulsify and cooled to prepare a liquid foundation.

Result of evaluation of the resulting liquid foundation was that spread upon application was good, touch was smooth and close adhering feeling was good. It also showed good effect for conditioning the skin texture, transparency and effect for making hair follicles imperceptible and the cosmetic membrane (film)upon finish was uniform and natural (refer to Table 3 which will be mentioned later).

### (Comparative Example 6)

A liquid foundation was prepared by the following method.

| | Components | Composition (mass %) |
|---|---|---|
| (A) | (1) Cyclomethicone | 12.0 |
| | (2) Volatile oil of emulsified type | 2.0 |
| | (3) Silicon-treated titanium oxide | 5.0 |
| | (4) Silicon-treated red iron oxide cloisonné | 0.7 |
| | (5) Silicon-treated yellow iron oxide | 0.2 |
| | (6) Silicon-treated black iron oxide | 0.1 |
| | (7) Silicon-treated talc | 6.0 |
| (B) | (8) Propyl paraben | 0.2 |
| | (9) Poloyxyethylene lauryl ether | 0.5 |
| (C) | (10) Volatile oil of emulsified type | 18.0 |
| | (11) Dimethylsilicone (50 cs) | 3.0 |
| | (12) Tocopherol acetate | 0.1 |
| | (13) Corn oil | 0.05 |
| (D) | (14) Methyl paraben | 0.2 |
| | (15) Propylene glycol | 8.0 |
| (E) | (16) Pure water | 41.45 |
| | (17) Sodium dehydroacetate | 0.3 |
| | (18) Pantothenyl alcohol | 0.2 |
| | (19) Sodium chloride | 2.0 |

With regard to the above components (A), pigment components (3) to (7) were mixed using a Henshel mixer and ground by an atomizer and other components (1) and (2) were added to the ground mixture followed by uniform mixing.

Each of the above components (B), (C), (D) and (E) was heated to dissolve at 60°C, the above uniformly mixed component (A), the above component (B) and the above component (C) were mixed and, similarly, the above component (D) and the above component (E) were mixed.

Aqueous layer component (a mixture of the components (D) and (E)) was added little by little to an oily layer component (a mixture of the components (A) to (C)) which was stirred using a homogenizer to emulsify and cooled to prepare a liquid foundation.

Result of evaluation of the resulting liquid foundation was that, although there was some smoothness of touch upon application, effect for conditioning the skin texture, transparency and effect for making pores imperceptible were inferior to the liquid foundation prepared in Example 4 (refer to Table 3 which will be mentioned later).

### (Sensory Evaluation Example 3)

In order to evaluate the liquid foundation prepared in each of the above Example 4 and Comparative Example 6, an sensory evaluation was conducted by 20 female panelists for spread, effect for conditioning the skin texture, transparency and effect for making hair follicles imperceptible. The result is shown in Table 3. The evaluation was conducted by a five-point method and the average mark was adopted.
- 5: very good
- 4: somewhat good
- 3: normal
- 2: somewhat inferior
- 1: much inferior

**[Table 3] Sensory Evaluation for Liquid Foundations**

| Samples | Spread | Skin Texture ^{*1} | Transparency | Hair follicles ^{*2} |
|---|---|---|---|---|
| Example 4 | 4.25 | 4.25 | 3.90 | 3.85 |
| Comp Ex 6 | 3.80 | 3.80 | 2.20 | 2.10 |

| | | | | |
|---|---|---|---|---|
| *1: Effect for conditioning the skin texture | | | | |
| *2: Effect for making hair follicles imperceptible | | | | |

From the above result, it is noted that, with regard to liquid foundation, product of the present invention is better than the conventional product.

### (Example 5)

The disintegrable porous spherical silica powder prepared in Manufacturing Example 6 was used and predetermined amounts of the following material components were compounded (mixed), mixed with stirring using a Henshel mixer and heated at 120°C for 7 hours to subject to a silicon treatment.

| Components | Composition (mass %) |
|---|---|
| (1) Dimethyl silicone oil (200 cs) | 14.0 |
| (2) Methyl hydrogen polysiloxane | 6.0 |
| (3) Silica powder prepared in Manufacturing Example 6 | 80.0 |

The silicon-treated disintegrable porous spherical silica powder prepared as above was used and predetermined amounts of the material components were compounded according to the following method to manufacture a skin care milky lotion.

Firstly, the material components (1) to (3) were mixed at room temperature and the material component (4) was added to the above material component mixture which was completely dissolved by heating at 90°C followed by mixing at 75°C.

After that, the material component (5) was added to the resulting material component mixture and mixed until the mixture became uniform.

The material components (6) to (8) were mixed at room temperature and completely dissolved at 75°C.

An aqueous-layer component (a mixture of the material components (6) to (8)) was added little by little to an oily-layer component (a mixture of the material components (1) to (5)) with stirring using a homogenizer to emulsify and cooled to prepare a skin care milky lotion.

With regard to the skin care milky lotion prepared as such, good spread upon application, smooth feeling, effect for conditioning the skin texture, transparency and effect for making pores imperceptible were noted.

| Components | Composition (mass %) |
|---|---|
| (1) Dextrin palmitate | 1,5 |
| (2) Glyceryl tri-2-ethylhexanoate | 4.7 |
| (3) Dimethyl polysiloxane copolyol | 1.85 |
| (4) Cyclomethicone | 18.5 |
| (5) Silicon-treated disintegrable porous spherical silica powder | 7.5 |
| (6) Sodium chloride | 0.46 |
| (7) Methyl paraben | 0.2 |
| (8) Pure water | 65.29 |

### ADVANTAGES OF THE INVENTION

In accordance with the present invention, there is provided a cosmetic, preferably a skin cosmetic, which has excellent functions of imparting transparency to the skin, conditioning skin texture and making wrinkles and hair follicles imperceptible. There is further provided a disintegrable porous spherical silica powder having a property of being disintegrated by pressure with finger, puff, etc., by abrasion and the like as a cosmetic powder therefor.

Consequently, the present invention is quite useful in an industrial view particularly in the cosmetic field.

## Claims

1. A cosmetic comprising a powder of porous spherical silica having an average particle size (based on volume) from 3.0 to 20 µm, a maximum particle size of 50 µm or smaller, and a pore volume from 1.5 to 3.0 cm³/g, wherein the powder is **characterized in that** the minimum value of dlog (storage elasticity)/dlog (shear stress) is -10 or more, when 40 g of squalane is added to 15 cm³ of apparent volume of said powder of porous spherical silica, the resulting paste is charged in thickness of 2 mm between parallel plates of 2.5 cm diameter, one of the plates is subjected to an angular vibration to another in a frequency of 2 Hz, dynamic viscoelasticity is measured by increasing an average shear stress between the plates from 10 Pa to 10 kPa and correlation between log (shear stress) versus log (storage elasticity) is measured.

2. The cosmetic according to claim 1, wherein said powder of porous spherical silica is comprised in amounts of 1 to 80% by mass.
